# EUROPEAN PATENT APPLICATION

(11) **EP 4 484 909 A1**
(43) Date of publication of application: **01.01.2025**
(21) Application number: 23766345.5
(22) Date of filing: 25.01.2023
(51) Int. Cl.: G01K 1/14, G01K 7/02, A61B 18/14

(54) **HIGH-FREQUENCY NEEDLE ELECTRODE WITH TEMPERATURE MEASUREMENT FUNCTION**

(30) Priority: 10.03.2022 JP 2022037292
(71) Applicant: Kabushiki Kaisha Top, Adachi-ku, Tokyo 120-0035 (JP)
(72) Inventor: MURATA Koji, Tokyo 120-0035 (JP); MIYAZAWA Yoshihiro, Tokyo 120-0035 (JP)
(74) Representative: Schön, Christoph
(86) International application number: PCT/JP2023/002180
(87) International publication number: WO 2023/171165

(57) **Abstract**

Provided is a high-frequency electrode needle with a temperature measurement function which can avoid a decrease in function while reducing a size of a needle base. Thermocouple wires (W1+, W1-) and compensation wires (W2+, W2-) are wound around each of a pair of winding portions (120) that are formed to locally protrude to an outside of a needle base (12) at a proximal portion (122), so that the thermocouple wires (W1+, W1-) and the compensation wires W2+ and W2- are electrically connected to each other, and the proximal portion (122), which is closer to a central axis (O) of the needle base (12) than a distal portion (121), is configured to be at least partially recessed or narrower than the distal portion (121).

## Description

### Technical Field

The present invention relates to a high-frequency electrode needle having a temperature measurement function, which can cauterize a specific tissue by discharging a high-frequency current and/or performing a nerve block by a pulse high frequency and/or administration of anesthetic solution from a needle tip portion.

### Background Art

A balloon catheter configured to prevent disconnection of a wire connected to a temperature sensor has been proposed (for example, see Patent Literature 1). Specifically, a copper wire for supplying a high-frequency voltage of a high-frequency generator to an electrode for high-frequency heating is connected to one end of a thermocouple sensor. A coil that separates the high-frequency signal is connected to the other end of the thermocouple sensor. The coil is incorporated in a catheter body. The copper wire and the coil are electrically connected to a thermometer.

With this configuration, the copper wire for the electrode is used as one wire of the thermocouple sensor, and a high frequency from a high-frequency generator is blocked by the coil connected to the other end of the thermocouple sensor, and a direct current temperature signal of the thermocouple sensor is output via the copper wire and the coil.

### Citation List

### Patent Literature

Patent Literature 1: Japanese Patent Application Laid-Open No. H05-293183

### Summary of Invention

### Technical Problem

However, in a case in which the thermocouple wire and the compensation wire are electrically connected to each other to form a compensation contact, when the connector is used, a size of the needle base is increased by the presence of the connector, and in a case where a plurality of high-frequency electrode needles is punctured into a body of a patient at a narrow interval, the plurality of high-frequency electrode needles may interfere with each other.

Therefore, an object of the present invention is to provide a high-frequency electrode needle with a temperature measurement function, which can avoid a decrease in function while reducing the size of the needle base.

### Solution to Problem

The present invention relates to a high-frequency electrode needle with a temperature measurement function (hereinafter, also referred to as a "high-frequency electrode needle" for simplification of the description), including: a tubular needle tube; a tubular needle base to which the needle tube is attached at a tip end portion; a thermocouple including a pair of thermocouple wires that are electrically connected to each other at a temperature measurement contact disposed at the tip end portion of the needle tube; a high-frequency wire disposed along an outside of the needle base and electrically connected to the needle tube; and a pair of compensation wires electrically connected to the pair of thermocouple wires, respectively.

In the high-frequency electrode needle of the present invention, the needle base is formed with a pair of winding portions that locally protrude outwards and have a proximal portion that is at least partially recessed or narrower than a distal portion about a central axis of the needle base, and each of the pair of thermocouple wires and each of the pair of compensation wires are wound around each of the pair of winding portions at the proximal portion to form a compensation contact.

With the high-frequency electrode needle of the configuration, the thermocouple wires and the compensation wires are wound around each of the pair of winding portions that are locally formed to protrude from the outside of the needle base at the proximal portion, so that the thermocouple wires and the compensation wires are electrically connected to each other. The wire may be a strand in at least a range in which an electrical contact is formed, and may be covered with an insulating material in a range in which the electrical contact is not formed. The proximal portion, which is closer to the central axis of the needle base than the distal portion, is at least partially recessed (or narrower) than the distal portion. Therefore, a misalignment of the wires wound around the winding portion at the proximal portion is hindered by the distal portion, and thus a situation in which the wires are detached from the winding portion is prevented. As a result, since the compensation contact is formed, a space required for reliable electrical connection between each of the pair of thermocouple wires and each of the pair of compensation wires is reduced, and thus the size of the needle base is reduced, as compared with a case in which the compensation contact is formed by the connector.

In the high-frequency electrode needle of the configuration, it is preferable that at least one recessed portion that is locally recessed is formed at the outside of the needle base, and at least one winding portion of the pair of winding portions is formed to locally protrude from a bottom surface of the at least one recessed portion.

With the high-frequency electrode needle of the configuration, an amount of protrusion of at least one winding portion from the outer surface (the outer surface around the recessed portion) of the needle base is reduced by at least an amount corresponding to at least partial accommodation of the at least one winding portion in the recessed portion of the needle base. As a result, the space required for the reliable electrical connection between each of the thermocouple wire and the compensation wire is minimized, and thus the needle base is further miniaturized.

In the high-frequency electrode needle of the configuration, it is preferable that the at least one winding portion is accommodated in the recessed portion of the needle base as a whole.

With the high-frequency electrode needle of the configuration, since at least one winding portion is accommodated in the recessed portion of the needle base as a whole, the space required for the reliable electrical connection between each of the thermocouple wire and the compensation wire is minimized, and thus the needle base is further miniaturized.

In the high-frequency electrode needle of the configuration, it is preferable that each of the pair of thermocouple wires and each of the pair of compensation wires are wound around each of the pair of winding portions at the proximal portion such that a free end surface of at least one of the thermocouple wire and the compensation wire faces a side opposite to the needle tube.

With the high-frequency electrode needle of the configuration, the thermocouple wire and the compensation wire are wound around the winding portion such that the free end surface (an end surface where the winding ends) of the thermocouple wire and/or the compensation wire face the side opposite to the needle tube. Therefore, when the needle base cover is attached to the needle base from the needle tip side, the free end surface of the wire wound around the winding portion makes contact with the needle base cover, and the end portion of the wire is removed from the winding portion, and thus, loosening of the winding of the wire can be avoided.

In the high-frequency electrode needle of the configuration, it is preferable that a groove is formed in the outside of the needle base along a longitudinal direction, and the high-frequency wire is disposed along the groove.

With the high-frequency electrode needle of the configuration, the size of the needle base including the high-frequency wire is reduced as compared with a case in which the high-frequency wire is exposed to the outside of the needle base while the interference and/or conduction with the thermocouple wire and/or the compensation wire is reliably prevented.

### Brief Description of Drawings

FIG. 1 is a configuration explanatory view of a high-frequency electrode needle as one embodiment of the present invention.
FIG. 2 is a detailed configuration explanatory view of an X1 part (tip end portion of a needle tube) of FIG. 1.
FIG. 3 is a detailed configuration explanatory view of the X1 part (tip end portion of the needle tube) of FIG. 1.
FIG. 4 is a detailed configuration explanatory view of an X2 part (needle base and winding portion) of FIG. 1.
FIG. 5A is another configuration explanatory view of the needle base and the winding portion.
FIG. 5B is another configuration explanatory view of the needle base and the winding portion.
FIG. 6 is an explanatory view related to an arrangement aspect of a high-frequency wire in the needle base.
FIG. 7 is a detailed configuration explanatory view of an X3 part (thermocouple connector) of FIG. 1.

### Description of Embodiments

### (Configuration of High-Frequency Electrode Needle)

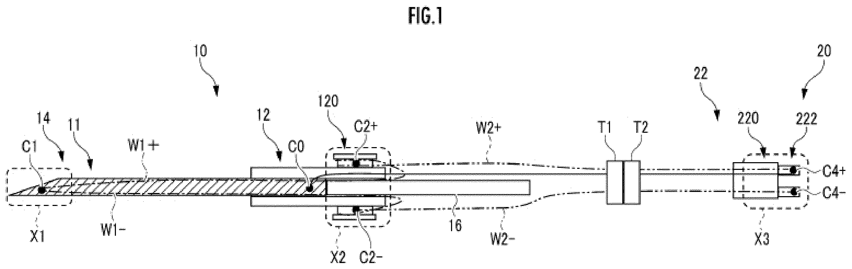

A high-frequency electrode needle as a first embodiment of the present invention, which is schematically illustrated in FIG. 1, includes a first unit 10 and a second unit 20.

As illustrated in FIG. 1, the first unit 10 includes a substantially cylindrical needle tube 11, a substantially cylindrical or substantially truncated conical cylindrical needle base 12 to which the needle tube 11 is attached at a tip end portion, a high-frequency wire W0 (see the solid line) electrically connected to the needle tube 11, a thermocouple 14 including a pair of thermocouple wires W1+ and W1- (see the one-dot chain line), a pair of compensation wires W2+ and W2- (see the two-dot chain line), and a first connector T1.

The needle tube 11 is attached to the needle base 12 in a state in which a rear end portion thereof is inserted from the tip end portion to an inside or hollow space of the needle base 12 and the inside of the needle tube 11 and the inside of the needle base 12 communicate with each other. A tip end portion of a chemical liquid tube 16 is inserted into the inside of the needle base 12 from the rear end portion. Accordingly, a chemical liquid is supplied from a chemical liquid supply device connected to the rear end portion of the chemical liquid tube 16 to the inside of the needle tube 11 through the chemical liquid tube 16, and the chemical liquid is discharged from the tip end portion of the needle tube 11.

The pair of thermocouple wires W1+ and W1- are electrically connected to each other at a temperature measurement contact C1 disposed at the tip end portion of the needle tube 11. The pair of thermocouple wires W1+ and W1- and the pair of compensation wires W2+ and W2- are electrically connected to each other at a pair of compensation contacts C2+ and C2-, respectively. As will be described below, a pair of winding portions 120 that locally protrude to the outside of the needle base 12 include the pair of compensation contacts C2+ and C2-. The high-frequency wire W0 and the pair of compensation wires W2+ and W2- may be collectively accommodated in a wiring tube (not illustrated) having insulating properties and flexibility. In this case, the wire may be led from a tip end portion of the wiring tube to the rear end portion of the needle base 12.

The needle tube 11 is formed of, for example, a conductive material such as stainless steel. The needle base 12 is formed of, for example, an insulating material such as an epoxy resin. The high-frequency wire W0 is formed of, for example, a copper wire (for example, ϕ0.18 mm) or an enameled wire. One thermocouple wire W1+ and one compensation wire W2+ are formed of, for example, a chromel wire (for example, ϕ0.1 mm). The other thermocouple wire W1- and the other compensation wire W2- are formed of, for example, an alumel wire (for example, ϕ0.1 mm). One compensation wire W2+ may be formed of a copper wire (for example, ϕ0.1 mm), and the other compensation wire W2- may be formed of a constantan wire (for example, ϕ0.1 mm). One thermocouple wire W1+ may be formed of a copper wire, and the other thermocouple wire W1- may be formed of a constantan wire. The high-frequency wire W0, the thermocouple wires W1+ and W1-, and/or the compensation wires W2+ and W2- may be covered with an insulating coating layer, except for a part constituting an electrical contact.

As illustrated in FIG. 1, the second unit 20 includes a high-frequency wire W0 (see the solid line), a pair of compensation wires W2+ and W2- (see the two-dot chain line), a thermocouple connector 22, and a second connector T2. The thermocouple connector 22 includes a body 220 and a pair of terminals 222 that protrude from the body 220. The pair of terminals 222 are inserted into a terminal receiving portion having a high-frequency power supply device (not illustrated) having a temperature measurement function to constitute reference temperature contacts C4+ and C4- electrically connected to the device (particularly, a temperature measurement device).

The first unit 10 and the second unit 20 are connected to each other in a removable manner via the first connector T1 and the second connector T2. The first connector T1 and the second connector T2 are connected to each other, so that the high-frequency wire W0 constituting the first unit 10, each of the pair of the compensation wires W2+ and W2- and the high-frequency wire W0 constituting the second unit 20, and each of the pair of the compensation wires W2+ and W2- are electrically connected to each other.

### (Configuration of Needle Tube)

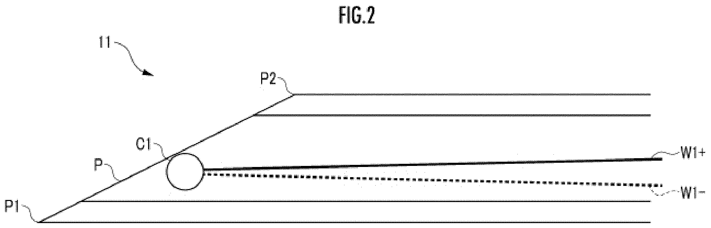

As illustrated in FIG. 2, the tip end portion of the needle tube 11 is formed with an inclined cutting edge surface P having a substantially planar shape and inclined with respect to a central axis of the needle tube 11. As illustrated in FIG. 2, the temperature measurement contact C1, at which the pair of thermocouple wires W1+ and W1-constituting the thermocouple 14 are electrically connected to each other, is disposed inside the needle tube 11 at a rear side of the inclined cutting edge surface P and midway between a tip end portion P1 and a rear end portion P2 of the inclined cutting edge surface P. The temperature measurement contact C1 may be disposed at a position corresponding to the rear end portion P2 of the inclined cutting edge surface P or the rear side of the rear end portion P2 of the inclined cutting edge surface P (for example, rear side by a distance equal to or less than 0.2 times a length of the inclined cutting edge surface P in an axial direction of the needle tube 11). The same applies to other embodiments.

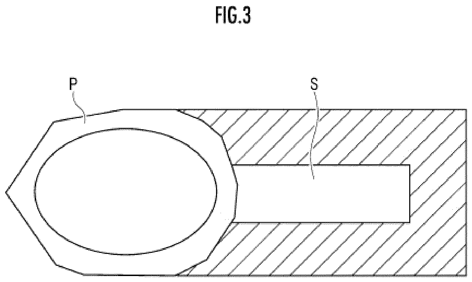

As illustrated in FIG. 3, the needle tube 11 is covered with an insulating material (for example, a fluorine coating material) except for the inclined cutting edge surface P and a local region S on an outer surface of the tip end portion. The local region S is a substantially rectangular region when viewed from a top, which extends in a range corresponding to the rear end portion P2 of the inclined cutting edge surface P in a circumferential direction of the needle tube 11 on the outer surface of the tip end portion, and extends in the longitudinal direction (a direction parallel to the central axis) of the needle tube 11. The shape of the region may be variously changed, in addition to the substantially rectangular shape, such as a substantially triangular shape, a substantially trapezoidal shape, a substantially circular shape, a substantially oval shape, a substantially elliptical shape, or a combination of these shapes.

In another embodiment, the tip end portion of the needle tube 11 may be covered with an insulating material except for at least a part of the inclined cutting edge surface P, or the tip end portion of the needle tube 11 may be covered with an insulating material except for the entire circumference of the inclined cutting edge surface P and the outer surface of the tip end portion of the needle tube 11.

### (Configuration of Needle Base)

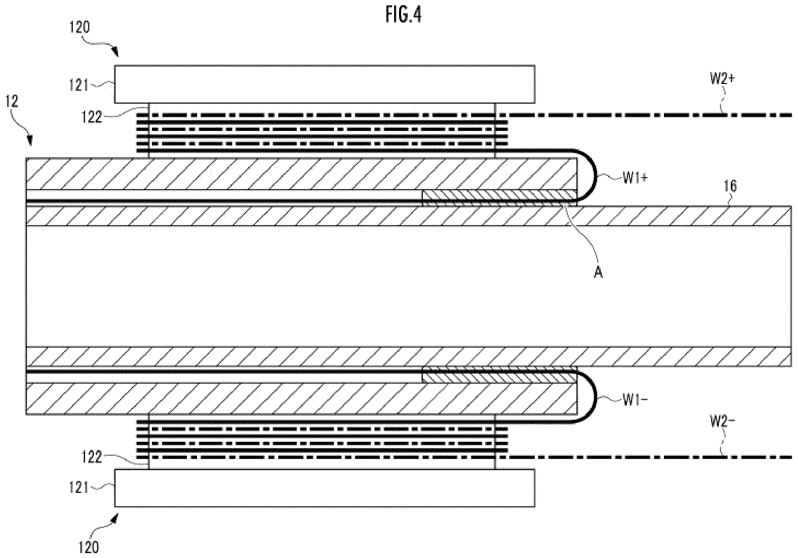

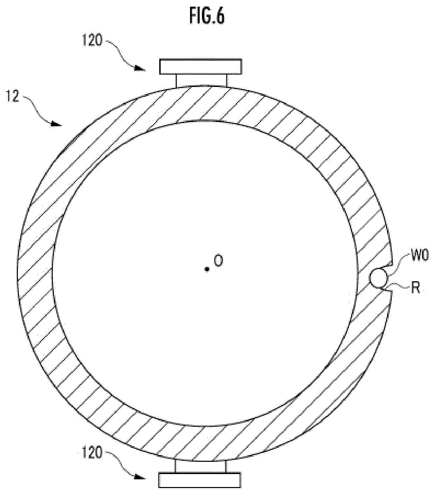

As illustrated in FIG. 4, the pair of winding portions 120 that locally protrude outward are formed on the needle base 12. As illustrated in FIG. 6, the pair of winding portions 120 are disposed to have two-time rotational symmetry with respect to a central axis O of the needle base 12. The pair of winding portions 120 may be disposed such that an azimuthal angle deviation with respect to the central axis O of the needle base 12 is not 180°, but is another value such as 45°, 60°, 75°, 90°, 120°, 135°, or 150°. The winding portion 120 is formed such that a proximal portion 122 is recessed or narrowed over the entire circumference with respect to the distal portion 121 of the needle base 12 about the central axis. For example, the proximal portion 122 may be formed in a substantially elongated cylindrical shape extending along the central axis of the needle base 12, and the distal portion 121 may be formed in a substantially elongated disc shape or a substantially elongated cylindrical shape that protrudes over the entire circumference of the proximal portion 122.

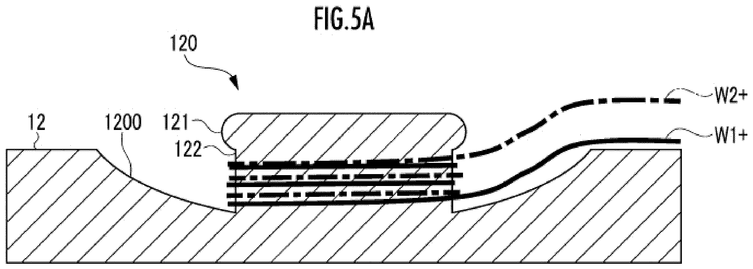

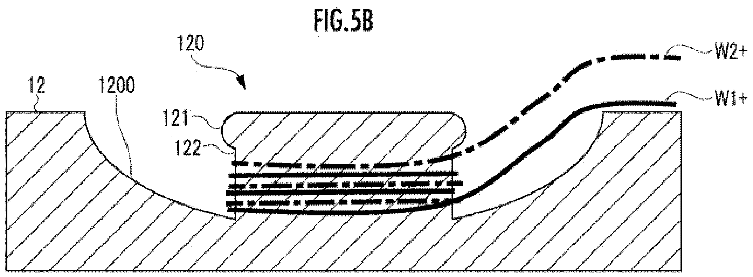

In the present embodiment, the winding portion 120 protrudes radially outward from the substantially cylindrical or substantially truncated conical cylindrical outer surface of the needle base 12. However, in another embodiment, as illustrated in FIG. 5A, a recessed portion 1200 that is locally recessed may be formed in the needle base 12 from the substantially cylindrical or substantially truncated conical cylindrical outer surface of the needle base 12, and the winding portion 120 may be configured to protrude radially outward from a bottom surface of the recessed portion 1200. In this case, the winding portion 120 may partially and radially protrude outside of the outer surface of the needle base 12 from the recessed portion of the needle base 12. In addition, as illustrated in FIG. 5B, the winding portion 120 may be entirely accommodated in the recessed portion of the needle base 12, for example, a tip end surface or a top surface of the winding portion 120 is disposed on the substantially same curved surface as the outer surface of the needle base 12.

As illustrated in FIG. 6, a groove R is formed in the outside of the needle base 12 along the longitudinal direction, and the high-frequency wire W0 is disposed along the groove R. For example, the high-frequency wire W0 is continuously provided on the inside of the needle base 12 through a slit formed in a side wall of the needle base 12 so as to extend from the tip end portion in the axial direction, and is electrically connected to the rear end portion of the needle tube 11 at a high-frequency contact C0 illustrated in FIG. 1 by soldering or the like.

In the present embodiment, an azimuthal angle of the groove R with respect to the central axis O of the needle base 12 is shifted by approximately 90° from an azimuthal angle of each of the pair of winding portions 120. The azimuthal angle of the groove R with respect to the central axis O of the needle base 12 may be optionally changed, for example, to 30°, 45°, 60°, or the like with respect to the azimuthal angle of one winding portion 120, within a range in which the wire wound around the one winding portion 120 and the high-frequency wire W0 disposed in the groove R do not interfere with each other or are not electrically conducted.

As illustrated in FIG. 4, one compensation contact C2+ is formed by winding one thermocouple wire W1+ and one compensation wire W2+ around one winding portion 120 in the proximal portion 122. As illustrated in FIG. 4, the other compensation contact C2-is formed by winding the other thermocouple wire W1- and the other compensation wire W2- around the other winding portion 120 in an overlapped manner at the proximal portion 122. The number of turns of each wire is, for example, "4", but may be the same or different from each other, and may be a number of turns other than "2" or "8" or the like.

It is preferable that the thermocouple wires W1+ and W1- and/or the compensation wires W2+ and W2- are wound around the winding portion 120 such that free end surfaces of the thermocouple wires W1+ and W 1- and/or the compensation wires W2+ and W2- are directed to a side opposite to the needle tube.

As illustrated in FIG. 4, each of the pair of thermocouple wires W1+ and W1- is wound around the winding portion 120 in this manner, and then wraps around from the rear end portion of the needle base 12 to be continuously provided to the inside of the needle base 12. As schematically shown in FIG. 4, each of the pair of thermocouple wires W1+ and W1-, which wraps around from the rear end portion of the needle base 12 to be continuously provided to the inside of the needle base 12, is fixed to the needle base 12 while being interposed between the inner surface of the needle base 12 and the outer surface of the chemical liquid tube 16. The needle base 12 and the chemical liquid tube 16 are liquid-tightly fixed by an adhesive layer A over the entire circumference. In FIG. 4, for easy understanding of the configuration, cross-sections of the needle base 12, the chemical liquid tube 16, and the adhesive layer A are illustrated.

A tubular needle base cover, which is guided from a side of the needle tube 11, may be attached to the outside of the needle base 12. The needle base 12 and the needle base cover attached to the needle base 12 may be fixed to each other by the adhesive.

### (Configuration of Thermocouple Connector)

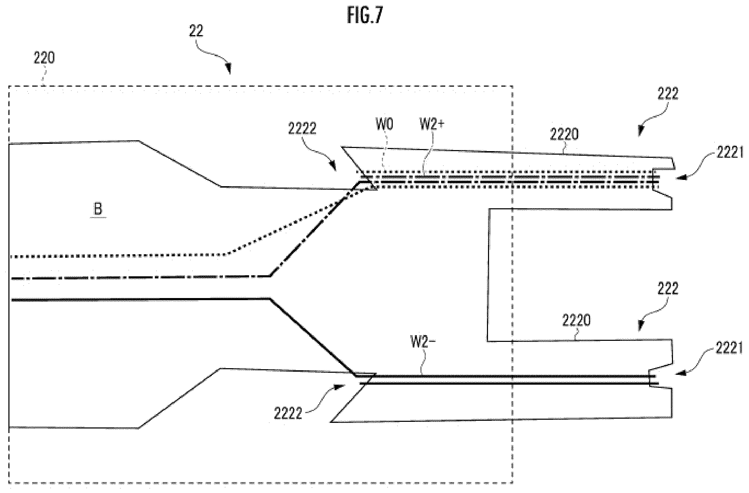

As schematically illustrated in FIG. 7, the thermocouple connector 22 includes a body 220 (housing) and a pair of terminals 222 that protrude from the body 220. The terminal 222 includes a pair of insulating base bodies 2220 which are spaced apart from each other in a lateral direction of a substantially rectangular substrate B, which is formed of an insulating material, from a tip end portion of the substrate B, and which protrude approximately parallel to the longitudinal direction of the substrate B. In the insulating base body 2220, a tip end recessed portion 2221, which is recessed in a substantially tongue piece shape in the longitudinal direction, is formed in a tip end portion that protrudes from the body 220. In the insulating base body 2220, a rear end recessed portion 2222, which is recessed in a substantially undercut shape in the longitudinal direction, is formed in the rear end portion accommodated in the body 220.

One terminal 222 provided on the upper side of FIG. 7 includes one insulating base body 2220, and one compensation wire W2+ and the high-frequency wire W0 (see dotted lines) that are wound around the one insulating base body 2220 so as not to overlap each other via the tip end recessed portion 2221 and the rear end recessed portion 2222. As illustrated in FIG. 7, the one compensation wire W2+ wound around the one insulating base body 2220 is interposed between the high-frequency wires W0 wound around the one insulating base body 2220 with a space therebetween.

The other terminal 222 on the lower side of FIG. 7 includes the other insulating base body 2220 and the other compensation wire W2- that is wound around the other insulating base body 2220 via the tip end recessed portion 2221 and the rear end recessed portion 2222.

### (Other Embodiments of Present Invention)

In the above embodiment, the first unit 10 and the second unit 20 are connected to each other in a removable manner via the first connector T1 and the second connector T2, but in other embodiments, the first unit 10 and the second unit 20 may be connected to each other in a non-removable manner via the first connector T1 and the second connector T2, or wires constituting each of the first unit 10 and the second unit 20 may be continuously provided.

The high-frequency electrode needle may include only the first unit 10. In this case, the first connector T1 may be omitted.

### Advantageous Effect of Invention

With the high-frequency electrode needle of the configuration, the thermocouple wires W1+ and W1- and the compensation wires W2+ and W2- are wound around each of the pair of winding portions 120 that are locally formed to protrude from the outside of the needle base 12 at the proximal portion 122, so that the thermocouple wires W1+ and W1- and the compensation wires W2+ and W2- are electrically connected to each other. The proximal portion 122, which is closer to the central axis O of the needle base 12 than the distal portion 121, is at least partially recessed (or narrower) than the distal portion 121. Therefore, a misalignment of the wires W1+, W1-, W2+, and W2- wound around the winding portion 120 at the proximal portion 122 is hindered by the distal portion 121, and thus a situation in which the wires W1+, W1-, W2+, and W2- are detached from the winding portion 120 is prevented.

As a result, since the compensation contact C2 is formed, a space required for reliable electrical connection between each of the pair of thermocouple wires W1+ and W1- and each of the pair of compensation wires W2+ and W2- is reduced, and thus the size of the needle base 12 is reduced, as compared with a case in which the compensation contact C2 is formed by the connector.

### Description of Reference Numerals

10: first unit
11: needle tube
12: needle base
120: winding portion
121: distal portion
122: proximal portion
14: thermocouple
16: chemical liquid tube
20: second unit
22: thermocouple connector
220: body (housing)
222: terminal
2220: insulating base body
2221: tip end recessed portion
2222: rear end recessed portion
C1: temperature measurement contact
C2: compensation contact
C4: reference temperature contact
P: inclined cutting edge surface
R: groove
S: local region (designated region)
T1: first connector
T2: second connector
W0: high-frequency wire
W1+, W1-: thermocouple wire
W2+, W2-: compensation wire

## Claims

1. A high-frequency electrode needle with a temperature measurement function, comprising:
a tubular needle tube;
a tubular needle base to which the needle tube is attached at a tip end portion;
a thermocouple configured by a pair of thermocouple wires that are electrically connected to each other at a temperature measurement contact disposed at a tip end portion of the needle tube;
a high-frequency wire disposed along an outside of the needle base and electrically connected to the needle tube; and
a pair of compensation wires electrically connected to the pair of thermocouple wires, respectively,
wherein the needle base is formed with a pair of winding portions that locally protrude outwards and have a proximal portion that is at least partially recessed or narrower than a distal portion with reference to a central axis of the needle base, and
each of the pair of thermocouple wires and each of the pair of compensation wires are wound around each of the pair of winding portions at the proximal portion to form a compensation contact.

2. The high-frequency electrode needle with a temperature measurement function according to claim 1,
wherein at least one recessed portion that is locally recessed is formed at the outside of the needle base, and
at least one winding portion of the pair of winding portions is formed to locally protrude from a bottom surface of the at least one recessed portion.

3. The high-frequency electrode needle with a temperature measurement function according to claim 2,
wherein the at least one winding portion is accommodated in the recessed portion of the needle base as a whole.

4. The high-frequency electrode needle with a temperature measurement function according to claim 1,
wherein each of the pair of thermocouple wires and each of the pair of compensation wires are wound around each of the pair of winding portions at the proximal portion such that a free end surface of at least one of the thermocouple wire and the compensation wire faces a side opposite to the needle tube.

5. The high-frequency electrode needle with a temperature measurement function according to claim 1,
wherein a groove is formed in the outside of the needle base along a longitudinal direction, and the high-frequency wire is disposed along the groove.
